# EUROPÄISCHE PATENTSCHRIFT

(11) **EP 1 179 326 B1**
(45) Veröffentlichungstag und Bekanntmachung des Hinweises auf die Patenterteilung: **23.11.2005**
(21) Anmeldenummer: 01117923.1
(22) Anmeldetag: 24.07.2001
(51) Int. Cl.: A61F 5/01, A61F 13/10

(54) **Handgelenkbandage**
Wrist bandage
Bandage pour poignet

(30) Priorität: 29.07.2000 DE 10037341
(43) Veröffentlichungstag der Anmeldung: 13.02.2002
(73) Patentinhaber: Beiersdorf AG, 20253 Hamburg (DE)
(72) Erfinder: Herzberg, Thorsten, 22419 Hamburg (DE)

(56) Entgegenhaltungen:
- EP-A- 0 071 212
- EP-A- 0 775 476
- EP-A- 0 970 669
- DE-A- 4 400 496
- FR-A- 2 181 157
- US-A- 3 238 939
- US-A- 5 649 900

## Beschreibung

Die Erfindung betrifft eine Bandage für das Handgelenk für Verletzungen des Handgelenkes, zum Beispiel Distorsionen, Torsionen der leichteren Art, Dehnungen und Gefügelockerungen im Bereich der Handwurzel und im Übergang zur Hand zu den Metacarpalen 1 bis 5 und beginnenden degenerativen Veränderungen des Daumen-Sattelgelenkes.

Orthopädische Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigem Material, zum Beispiel aus Neopren, Gewirke, oder Geweben.
Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.
Das Zusammenfügen kann durch Nähen, Kleben oder andere übliche Verfahren erfolgen.

Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Verbände oder Bandagen des Handgelenks werden bei Distorsionen, Kontusionen oder Zerrungen der ulnaren und radialen Bänder eingesetzt. Aber auch bei Fissuren der Metakarpalknochen können diese den Heilungsprozeß unterstützen. Schließlich können durch eine entsprechende Ruhigstellung des Handgelenks Reizzustände der Metakarpalgelenke bis zum völligen Verschwinden verringert werden.

Aus der EP 0 775 476 A ist eine beidhändig zu tragende Bandage für das Handgelenk bekannt, die ein anatomisch gerecht geformtes, flexibles Trägermaterial einsetzt, auf das zwei Taschen jeweils im seitlichen Kantenbereich aufgenäht sind, die zur Aufnahme einer Schiene dienen. Mit Hilfe mehrerer Zügel wird die Bandage um das Handgelenk angelegt und fixiert.

Aus EP-A-0 970 669 ist ein weiteres Beispiel einer Handgelenks bandage bekannt, die eine Dammenöffnung zur Aufnahme des Patientendammens sowie einem äußeren und zentralen Gurt aufwest.

Der Erfindung liegt die Aufgabe zugrunde, eine Bandage zu konzipieren, die ein hohes Maß an Funktionalität mit propriozeptiver Wirkung aufweist, gleichzeitig aber einfach und unkompliziert für den Patienten anzulegen ist und durch den Verzicht von starren Elementen ein hohes Maß an Dynamik aufweist. Außerdem soll die Bandage kostengünstig herzustellen sein.

Diese Aufgabe wird durch die gemäß Hauptanspruch gekennzeichnete Bandage gelöst. Gegenstand der Unteransprüche sind dabei vorteilhafte Weiterbildungen der Bandage.

Demgemäß beschreibt die Erfindung eine Bandage für das Handgelenk mit einem ersten gewinkelten Streifen, der aus zwei aufeinander in einem spitzen Winkel zulaufenden Schenkeln besteht, deren innere Kanten zumindest abschnittsweise, bevorzugt über die gesamte Länge der Kanten, miteinander verbunden, insbesondere vernäht, sind, einem zweiten länglichen Streifen, der am zweiten Schenkel des gewinkelten Streifens ansetzt, und der im distalen Unterarmbereich im Übergang zur Handgelenkwurzel die Handgelenkwurzel zirkulär umfaßt.
Im Übergangsbereich zwischen den Schenkeln setzt im ersten Schenkel ein im wesentlichen runder Einschnitt an, der sich bis in den zweiten Schenkel erstreckt und der bei angelegter Bandage zur Aufnahme des Daumens dient.

In einer ersten vorteilhaften Ausführungsform der Bandage beträgt der Winkel α zwischen den Schenkeln des gewinkelten Streifens zwischen 20° bis 45°, insbesondere 25° bis 40°.

In einer weiteren vorteilhaften Ausführungsform der Bandage ist ein Verbindungsstreifen vorhanden, der zum einen am länglichen Streifen und zum anderen am ersten Schenkel des gewinkelten Streifens ansetzt.

Der längliche Streifen der Bandage umfaßt im distalen Unterarmbereich im Übergang zur Handgelenkwurzel die Handgelenkwurzel zirkulär, und zwar vorzugsweise verschließbar. Zusätzlich wird der Daumen in einer Art Schlaufe mit einer laschenartigen Anstützung versehen.

Von der Daumenseite zur Handwurzel hin ist der Zuschnitt der Bandage durch eine Naht verbunden. Diese Naht sorgt durch ihre Gestaltung für eine hervorragende anatomische Paßform und Anstützung des Daumens der Bandage im Daumen/Handwurzetbereich.

Zuzüglich wird durch die bevorzugte Integration eines Verbindungsstückes, eines bevorzugt elastischen textilen Teiles auf die Innenseite der Bandage, ein einfaches Anlegen und sicheres Positionieren im offenen Zustand der Bandage mit nur einer Hand ermöglicht.

Aus biomechanischer Sicht hat es sich als besonders vorteilhaft erwiesen, wenn in der Bandage die Aussparung für den Daumen in U-förmiger Form ein bestimmtes Höhen- und Breitenverhältnis aufweist, da nur so eine Anstützung des Daumens und eine Daumenschlaufe verwirklicht werden kann. Die Höhe H der Daumenschlinge in Relation zur Breite B weist vorzugsweise ein Verhältnis von 1 zu 1,0 bis 2,2, besonders vorzugsweise 1 zu 1,7, auf.

In einer weiteren bevorzugten Ausführungsform ist die Bandage aus einem mindestens beidseitig kaschierten Material gefertigt, das ein klettfähiges Velour zur Außenseite oder das partiell befestigte klettbare Stücke zur Außenseite und ein hautfreundliches textiles Gewebe zur Innenseite aufweist.
Ebenfalls können Abstandsgewebe mit entsprechender Kaschierung oder im Falle einer technischen, maschinellen Ausrüstung auch ohne Kaschierung verwendet werden.
Derartige Abstandsgewebe werden in der EP 0 071 212 B1 offenbart. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasem, die über die Fläche des Schichtkörpers verteilt durch die Partikelschicht hindurchgenadelt sind und die Deckschicht und die Unterlagsschicht untereinander verbinden. Als zusätzliches, aber nicht erforderliches Merkmal sind gemäß EP 0 071 212 B1 in den Haltefasem Partikel aus inerten Gesteinspartikeln, wie zum Beispiel Sand, Kies oder dergleichen, vorhanden.
Die durch die Partikelschicht hindurchgenadelten Haltefasem halten die Deckschicht und die Unterlagsschicht in einem Abstand voneinander, und sie sind mit der Deckschicht und der Unterlagsschicht verbunden.
Abstandsgewebe oder -gewirke sind u. a. in zwei Artikeln beschrieben, und zwar
einem Artikel aus der Fachzeitschrift "kettenwirk-praxis 3/93", 1993, Seiten 59 bis 63 "Raschelgewirkte Abstandsgewirke"
und
einem Artikel aus der Fachzeitschrift "kettenwirk-praxis 1/94", 1994, Seiten 73 bis 76 "Raschelgewirkte Abstandsgewirke"
auf deren Inhalt hiermit Bezug genommen wird und deren Inhalt Teil dieser Offenbarung und Erfindung wird.

Weiterhin bevorzugt werden als Material ein Neoprenschaum, ein Polyurethanschaum oder ein Polyesterschaum verwendet, die zur besseren Belüftung bevorzugt perforiert sind.

In einer weiteren bevorzugten Ausführungsform der Bandage weist das Material eine Elastizität von 30 bis 150 % in der X-Achse und von 5 bis 70 % in der Y-Achse auf, wobei die X-Achse im angelegten Zustand zirkulär um den Unterarm beziehungsweise um das Handgelenk verläuft und die Y-Achse ebenfalls im angelegten Zustand in Unterarmlängsrichtung, also longitudinal.

In einer weiteren bevorzugten Ausführungsform der Bandage weist das Material für das Verbindungsteil eine Elastizität von 50 bis 200 % in der X-Achse und von 0 bis 50 % in der Y-Achse auf, wobei die X-Achse im angelegten Zustand zirkulär um den Unterarm beziehungsweise um das Handgelenk verläuft und die Y-Achse ebenfalls im angelegten Zustand in Unterarmlängsrichtung, also longitudinal.

Schließlich stellt es eine hervorragende Ausgestaltung der Bandage dar, wenn das Unterarmteil, sprich der längliche Streifen, einen oder mehrere Klettverschlüsse oder Druckknöpfe aufweisen.

Damit weicht die erfindungsgemäße Bandage deutlich von herkömmlichen Bandagen ab, die im allgemeinen lediglich das Handgelenk oder teilweise auch nur das distale Ende der Elle und Speiche umfassen.

Die Bandage schränkt kaum das physiologische Bewegungsumfeld der Hand ein und läßt neben dem Adaptivgriff auch den Spritzgriff zu. Durch die zirkuläre Kompression der Bandage im Handwurzelbereich wird ein Gefühl der erhöhten Gelenkfestigkeit gegeben. Dadurch werden ansonsten schmerzhafte Bewegungen wieder möglich, welche zu einer insgesamt besseren Durchblutung und Ernährung des Kapsel-Bandapparates führt.

Die Erfindung wird anhand von sechs schematischen Zeichnungen eines Ausführungsbeispiels näher erläutert, ohne damit die Erfindung unnötig einschränken zu wollen.

Es zeigen
- Figur 1: den Zuschnitt einer besonders vorteilhaften Ausführungsform der Bandage,
- Figur 2: Details zum Einschnitt in der Bandage, der bei angelegter Bandage zur Aufnahme des Daumens dient,
- Figur 3: die halb angelegte Bandage mit Ansicht von dorsal,
- Figur 4: die halb angelegte Bandage mit Ansicht von medial,
- Figur 5: die angelegte Bandage mit Ansicht von dorsal und
- Figur 6: die angelegte Bandage mit Ansicht von medial.

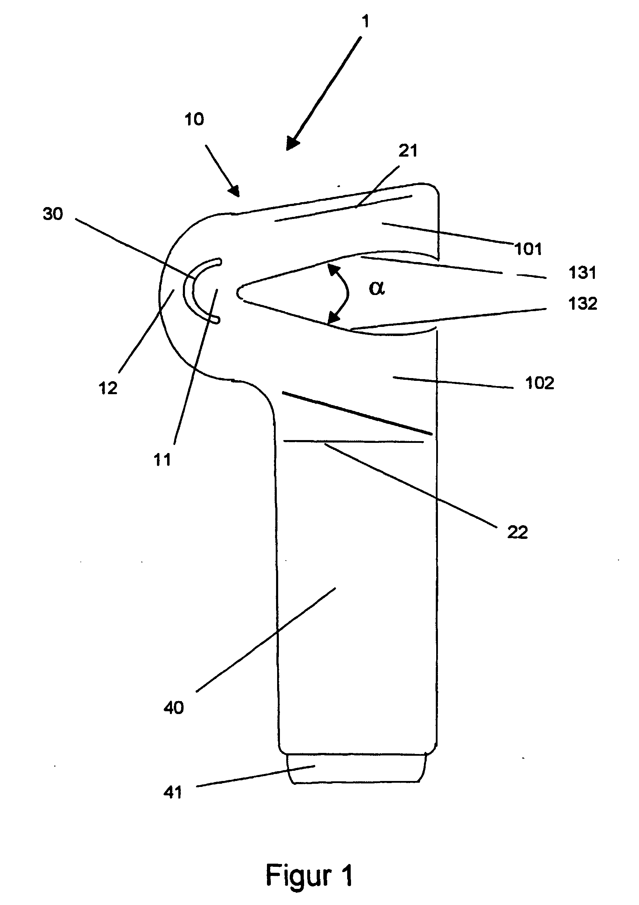

In der Figur 1 ist der Zuschnitt einer erfindungsgemäßen Bandage für das Handgelenk dargestellt. Die Bandage setzt sich aus einem ersten gewinkelten Streifen 10, der seinerseits aus zwei aufeinander in einem spitzen Winkel zulaufenden Schenkeln 101, 102 besteht, und einem zweiten länglichen Streifen 40 zusammen, der am zweiten Schenkel 102 des gewinkelten Streifens 10 ansetzt.

Im Übergangsbereich zwischen den Schenkeln 101, 102 setzt im ersten Schenkel 101 ein im wesentlichen runder Einschnitt 30 an, der sich bis in den zweiten Schenkel 102 erstreckt und der bei angelegter Bandage 1 zur Aufnahme des Daumens dient.
Somit bildet der Übergangsbereich zwischen den Schenkeln 101, 102 zum einen eine Daumenschlaufe 12, die den Daumen an der Wurzel umschließt, und zum anderen eine Daumenanstützung 11, die die Bewegungsfähigkeit des Daumens einschränkt und gleichzeitig eine Unterstützung für den Daumen bietet.

Der zweite Streifen 40 umfaßt im distalen Unterarmbereich im Übergang zur Handgelenkwurzel die Handgelenkwurzel zirkulär. Am Ende des zweiten Streifen 40 ist eine Lasche 41 vorhanden, die insbesondere mittels einer Klettvorrichtung auf dem Streifen 40 selbst haftet, um den Streifen 40 zu fixieren.

Die Bandage 1 weist zwischen den Schenkeln 101, 102 des gewinkelten Streifens 10 insbesondere einen Winkel α zwischen 20° bis 45° auf, hier ist ein Winkel von 35° vorhanden.

Zur Herstellung der Bandage 1 werden die inneren Kanten 131, 132 der beiden Schenkel 101, 102 zumindest abschnittsweise, bevorzugt über die gesamte Länge der Kanten 131, 132, miteinander verbunden. Bevorzugt erfolgt dies durch Nähen. Die Naht 13 erstreckt sich dabei ausgehend von den Querkanten der Schenkel 101, 102, die dem Übergangsbereich gegenüber liegen, bis an den Übergangsbereich selbst.

In der hier gezeigten vorteilhaften Ausführungsform der Bandage 1 ist ein Verbindungsstreifen 20 vorhanden, der zum einen am länglichen Streifen 40 und zum anderen am ersten Schenkel 101 des gewinkelten Streifens 10 ansetzt. Vorzugsweise erfolgt die Befestigung des Verbindungsstreifens ebenfalls durch Vernähen an den dargestellten Nähten 21, 22.

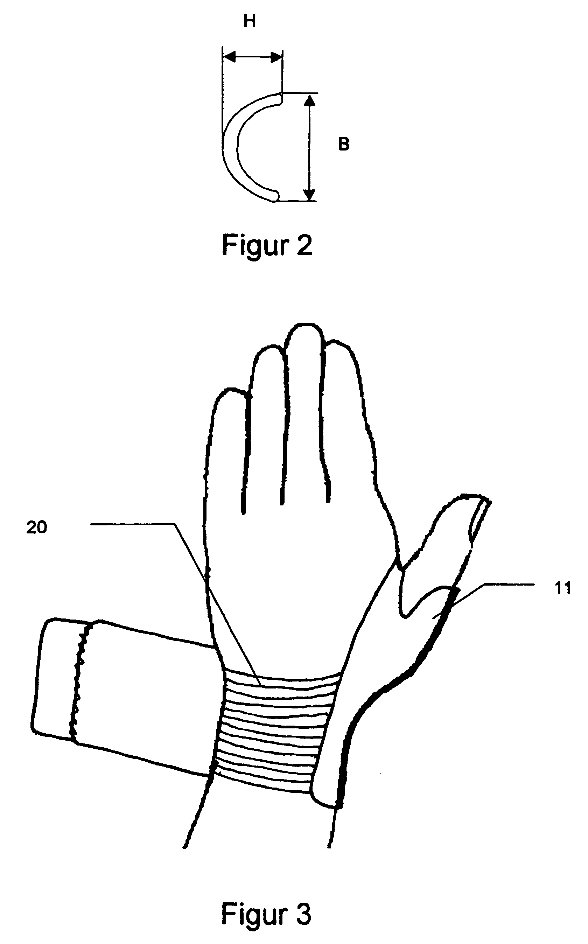

Die Figur 2 zeigt Details zum Einschnitt 30 in der Bandage 1, der bei angelegter Bandage 1 zur Aufnahme des Daumens dient.
Die Aussparung 30 ist vorzugsweise U-förmig ausgeformt, und zwar bei einem bestimmten Höhen- und Breitenverhältnis. Somit ist eine Anstützung des Daumens gewährleistet, und kann eine Daumenschlaufe verwirklicht werden. Die Höhe H der Daumenschlinge in Relation zur Breite B weist vorzugsweise ein Verhältnis von 1 zu 1,0 bis 2,2, besonders vorzugsweise 1 zu 1,7, auf.

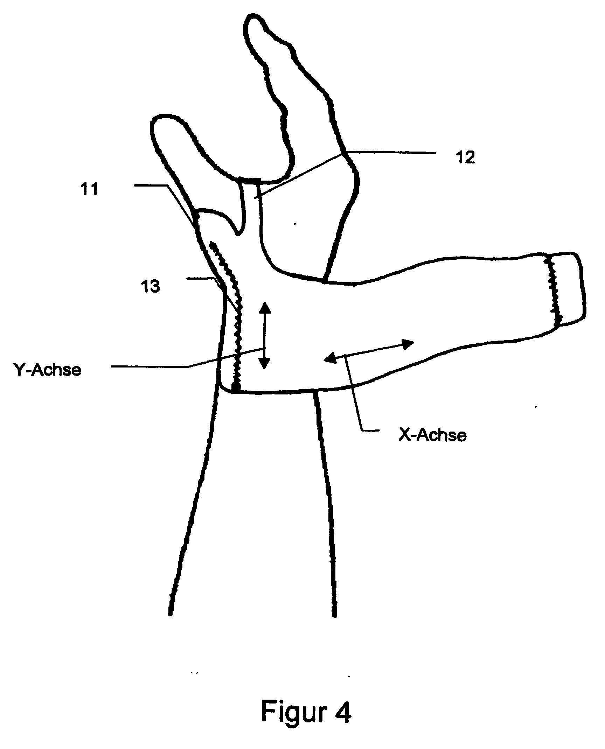

Die Figuren 3 und 4 stellen die jeweils halb angelegte Bandage mit Ansicht von dorsal beziehungsweise von medial dar.
Durch die Naht 13 sind die zwei Schenkel 101, 102 des gewinkelten Streifens 10 miteinander verbunden, und zwar derart, daß sich die Daumenabstützung 11 sowie die Daumenschlaufe 12 bilden.
Durch die bevorzugte Integration des Verbindungsstückes 20, das vorteilhaft ein elastisches textiles Teil ist, wird das einfache Anlegen und sicheres Positionieren im offenen Zustand der Bandage 1 mit nur einer Hand ermöglicht.

Das Material der Bandage weist eine Elastizität von 30 bis 150 % in der X-Achse und von 5 bis 70 % in der Y-Achse auf, wobei die X-Achse im angelegten Zustand zirkulär um den Unterarm beziehungsweise um das Handgelenk verläuft und die Y-Achse ebenfalls im angelegten Zustand in Unterarmlängsrichtung, also longitudinal (siehe Figur 4).

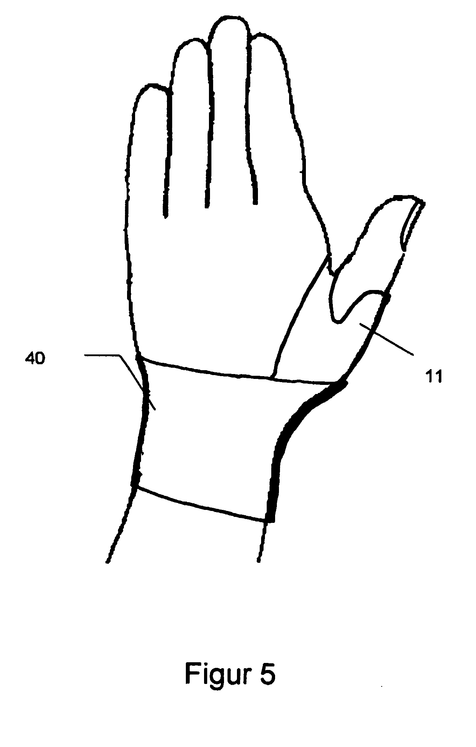

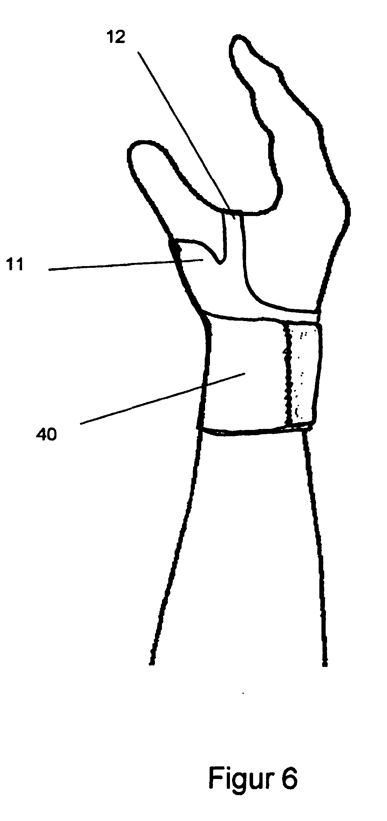

Die Figuren 5 und 6 zeigen schließlich die angelegte Bandage 1 mit Ansicht von dorsal und medial.
Der zweite Streifen 40 umfaßt die Handgelenkwurzel zirkulär. Mittels eines bevorzugten Klettverschlusses auf der Lasche 41 wird der Streifen 40 auf dem Streifen 40 selbst fixiert.

## Patentansprüche

1. Bandage(1) für das Handgelenk mit einem ersten gewinkelten Streifen (10), der aus zwei aufeinander in einem spitzen Winkel zulaufenden Schenkeln (101, 102) besteht, deren innere Kanten (131, 132) zumindest abschnittsweise miteinander verbunden sind, einem zweiten länglichen Streifer (40), der am zweiten Schenkel (102) des gewinkelten Streifens (10) ansetzt, wobei im Übergangsbereich zwischen den Schenkeln (101, 102) im ersten Schenkel (101) ein im wesentlichen runder Einschnitt (30) ansetzt, der sich bis in den zweiten Schenkel (102) erstreckt und der bei angelegter Bandage (1) zur Aufnahme des Daumens dient, und wobei der zweiten länglichen Streifen (40) im distalen Unterarmbereich im Übergang zur Handgelenkwurzel die Handgelenkwurzel zirkulär umfaßt.

2. Bandage nach Anspruch 1, **dadurch gekennzeichnet, daß** der Winkel (α) zwischen den Schenkeln (101,102) des gewinkelten Streifens (10) zwischen 20° bis 45° beträgt, insbesondere 25° bis 40°.

3. Bandage nach den Ansprüchen 1 und 2, **dadurch gekennzeichnet, daß** die Höhe (H) der Daumenschlinge in Relation zur Breite (B) ein Verhältnis von 1 zu 1,0 bis 2,2, vorzugsweise 1 zu 1,7, aufweist.

4. Bandage nach den Ansprüchen 1 bis 3, **dadurch gekennzeichnet, daß** ein Verbindungsstreifen (20) vorhanden ist, der zum einen am länglichen Streifen (40) und zum anderen am ersten Schenkel (101) des gewinkelten Streifens (10) ansetzt.

5. Bandage nach den Ansprüchen 1 bis 4, **dadurch gekennzeichnet, daß** die Bandage (1) aus einem mindestens beidseitig kaschierten Material gefertigt ist, das ein klettfähiges Velour zur Außenseite und ein hautfreundliches textiles Gewebe zur Innenseite aufweist, oder aus einem Abstandsgewebe.

6. Bandage nach Anspruch 5, **dadurch gekennzeichnet, daß** als Material ein Neoprenschaum, ein Polyurethanschaum oder ein Polyesterschaum verwendet werden, die bevorzugt perforiert sind.

7. Bandage nach den Ansprüchen 1 bis 6, **dadurch gekennzeichnet, daß** der längliche Streifen zumindest einen Klettverschluß oder Druckknöpfe aufweist.

## Claims

1. Wrist bandage (1) with a first angled strip (10) which consists of two branches (101, 102) which run together at an acute angle and whose inner edges (131, 132) are connected to one another at least in sections, and with a second elongate strip (40) which is attached to the second branch (102) of the angled strip (10), and in the transition area between the branches (101, 102) a substantially round incision (30) is made which starts in the first branch (101) and extends into the second branch (102) and, when the bandage (1) is applied, serves to receive the thumb and the second elongate strip (40), in the distal forearm area, at the transition to the carpal area, wraps round the carpal area in a circle.

2. Bandage according to Claim 1, **characterized in that** the angle (α) between the branches (101, 102) of the angled strip (10) is between 20° and 45°, in particular between 25° and 40°.

3. Bandage according to Claims 1 and 2, **characterized in that** the height (H) of the thumb loop in relation to the width (B) has a ratio of 1 to 1.0 to 2.2, preferably 1 to 1.7.

4. Bandage according to Claims 1 to 3, **characterized in that** a connection strip (20) is provided which is attached at one end to the elongate strip (40) and is attached at the other end to the first branch (101) of the angled strip (10).

5. Bandage according to Claims 1 to 4, **characterized in that** the bandage (1) is made of a material which is laminated at least on both sides and which has a velcro-like velour on the outside and a skin-compatible textile fabric on the inside, or of a spacer fabric.

6. Bandage according to Claim 5, **characterized in that** the materials used are neoprene foam, polyurethane foam or polyester foam, which are preferably perforated.

7. Bandage according to Claims 1 to 6, **characterized in that** the elongate strip has at least one velcro closure or press studs.

## Revendications

1. Bandage (1) pour le poignet, avec une première bande coudée (10) qui est constituée de deux branches (101, 102) qui forment l'une avec l'autre un angle aigu et dont au moins certaines parties des bords intérieurs (131, 132) sont reliées l'une à l'autre, une deuxième bande allongée (40) qui se raccorde à la deuxième branche (102) de la bande coudée (10), une découpe (30) essentiellement ronde qui s'étend jusque dans la deuxième branche (102) et qui sert à recevoir le pouce lorsque le bandage (1) est placé étant agencée dans la première branche (101) dans la zone de transition entre les branches (101, 102), la deuxième bande allongée (40) entourant en cercle la base du poignet dans la zone distale de l'avant-bras située à la transition avec la base du poignet.

2. Bandage selon la revendication 1, **caractérisé en ce que** l'angle (α) entre les branches (101, 102) de la bande coudée (10) est compris entre 20° et 45° et en particulier entre 25° et 40°.

3. Bandage selon les revendications 1 et 2, **caractérisé en ce que** la hauteur (H) de la boucle de pouce présente par rapport à la largeur (B) un rapport compris entre 1 et 1,0 à 2,2 et de préférence entre 1 et 1,7.

4. Bandage selon les revendications 1 à 3, **caractérisé en ce qu'**il présente une bande de liaison (20) qui se raccorde d'une part à la bande allongée (40) et d'autre part à la première branche (101) de la bande coudée (10).

5. Bandage selon les revendications 1 à 4, **caractérisé en ce que** le bandage (1) est réalisé en un matériau doublé au moins des deux côtés, qui présente sur son côté extérieur un velours apte à s'accrocher et sur son côté intérieur un tissu textile compatible avec la peau, ou en un tissu d'écartement.

6. Bandage selon la revendication 5, **caractérisé en ce que** comme matériau, on utilise une mousse de néoprène, une mousse de polyuréthane ou une mousse de polyester qui est de préférence perforée.

7. Bandage selon les revendications 1 à 6, **caractérisé en ce que** la bande allongée présente au moins une fermeture à accrochage ou des boutons-poussoirs.
